# EUROPÄISCHE PATENTANMELDUNG

(11) **EP 0 933 065 A1**
(43) Veröffentlichungstag der Anmeldung: **04.08.1999**
(21) Anmeldenummer: 98810076.4
(22) Anmeldetag: 02.02.1998
(51) Int. Cl.: A61B 17/70

(54) **Schwenkbares Befestigungssystem an einer Knochenschraube**

(71) Anmelder: Sulzer Orthopädie AG, 6340 Baar (CH)
(72) Erfinder: Studer, Armin, 6312 Steinhausen (CH); Donno, Cosimo, 8400 Winterthur (CH)
(74) Vertreter: Sulzer Management AG

(57) **Zusammenfassung**

Mit der Erfindung ist ein schwenkbares Befestigungssystem zwischen einer Knochenschraube (1) mit einem kugelförmigen Kopfteil (2) und einem Aufnahmeteil (3) gezeigt. Das Aufnahmeteil besitzt eine Durchgangsbohrung (4) mit einer Schulter am Ende, die das Kopfteil (2) auf seiner Unterseite umfasst und einen Anschlag bildet, wenn das Kopfteil (2) in einer einstellbaren Schwenkstellung (8) durch eine Klemmschraube (7) angepresst wird. Das Kopfteil (2) ist als separates Teil (9) ausgeführt, welches mit der Knochenschraube (1) verschraubbar ist, um das Aufnahmeteil (3) nach dem Implantieren der Knochenschraube (1) aufsetzen und mit dieser verbinden zu können.

## Beschreibung

Die Erfindung handelt von einem schwenkbaren Befestigungssystem zwischen einer Knochenschraube mit einem kugelförmigen Kopfteil und einem Aufnahmeteil, welches am Ende einer Durchgangsbohrung das Kopfteil mit einer Schulter an dessen Unterseite umfasst und welches eine Klemmschraube in der Durchgangsbohrung aufnimmt, mit der das Kopfteil in einer wählbaren Schwenkstellung gegen die Schulter anpressbar ist.

In der EP-A-0 614 649 wird eine Knochenschraube gezeigt, die vor dem Eindrehen in ein Implantat durch ein Aufnahmeteil gesteckt werden muss, welches beim Eindrehen hinderlich ist. Gerade bei Wirbelkorrekturen werden mehrere solcher Knochenschrauben, die in verschiedenen Wirbeln eingeschraubt sind, über Verbindungsstangen und Aufnahmeteile miteinander verbunden. Das Aufnahmeteil ist turmartig ausgeführt und nimmt den Kugelkopf der Knochenschraube in sich auf. Die Knochenschraube ist an einer Gegenschale mit ihrem Kugelkopf schwenkbar gelagert und kann entweder direkt durch eine Klemmschraube oder indirekt über Zwischenglieder wie Verbindungsstange und Druckscheibe durch eine Klemmschraube auf das Aufnahmeteil festgesetzt werden.

Ähnliche Überlegungen zeigt die DE-A-195 42 116 für ein plattenförmiges Aufnahmeteil, welches vor dem Festsetzen von Kugelflächen des Schraubenkopfes, um den gemeinsamen Mittelpunkt dieser Kugelflächen schwenkbar ist.

Beiden Anordnungen ist gemeinsam, dass die Knochenschraube durch das Aufnahmeteil hindurch eingeschraubt werden muss.

Aufgabe der Erfindung ist es, diesem Umstand abzuhelfen. Diese Aufgabe wird dadurch gelöst, dass das kugelförmige Kopfteil als ein separates Teil ausgeführt ist, welches mit der Knochenschraube verschraubbar ist, um das Aufnahmeteil nach dem Implantieren der Knochenschraube aufsetzen und mit dieser verbinden zu können.

Die Anordnung hat den Vorteil, dass die Knochenschraube beispielsweise als Pedikelschraube beim Einschrauben frei zugänglich ist. Ein Operateur kann während dem Eindrehen Lage und Sitz der Schraube, sowie den Zustand vom Knochengewebe überprüfen. Bei der Verbindung von mehreren Pedikelschrauben können die Aufnahmeteile provisorisch auf vorgebogenen Verbindungsstangen befestigt und durch Aufsetzen an den Pedikelschrauben in ihrer Lage korrigiert werden, um anschliessend separate Kugelköpfe auf den Pedikelschrauben zu befestigen und Klemmschrauben lose einzudrehen. Die provisorisch festgesetzten Verbindungsstangen können noch einmal in ihrer Befestigung zum Aufnahmeteil gelockert werden und anschliessend können alle Verbindungen gleichmässig angezogen werden. Dieses Vorgehen empfiehlt sich, wenn die Wirbel zueinander ausgerichtet sind. Wenn jedoch Verschiebungen der Wirbel zueinander notwendig sind, wird auf eine Lockerung der auf Verbindungsstangen ausgerichteten Aufnahmeteile bewusst verzichtet. Ein weiterer Vorteil ist eine flache Bauweise vom Aufnahmeteil und die Möglichkeit, bestehende Standard-Pedikelschrauben zu verwenden, um zu einem vernünftigen Schwenkbereich und zu einem erleichterten Einsetzen der Pedikelschrauben zu kommen.

Vorteilhafte Weiterbildungen der Erfindung sind in den Ansprüchen 2 bis 11 aufgezeigt.

Die Ausführung des separaten kugelförmigen Kopfteils als Schraubenmutter, die mit einem schraubenförmigen Fortsatz der Knochenschraube verschraubbar ist erweitert den Anwendungsbereich bei Standard-Pedikelschrauben. Dadurch, dass diese Schraubenmutter durch Schlitze geschwächt wird, setzt sie sich beim Zusammenpressen auf ihrem Gegengewinde fest. Die Knochenschraube muss daher nicht notwendigerweise einen Anschlag auf ihrem schraubenförmigen Fortsatz für die kugelförmige Schraubenmutter aufweisen. Die Schraubenmutter kann daher auch in ihrer Höhe verstellt und anschliessend durch Zusammenpressen auf dem Gegengewinde festgesetzt werden. Eingriffsflächen in Form von Einbuchtungen erleichtern die Höhenverstellung der kugelförmigen Schraubenmutter. Die Schulter des Aufnahmeteils und die Anpressfläche der Klemmschraube sind als konische Flächen ausgebildet, deren halber Konuswinkel α in bestimmten Grenzen 8° < α < 25° liegen sollte, um eine gute Klemmwirkung und geringen Platzbedarf zu erreichen. Mit den konischen Flächen sind gegenüber einer Kugelfläche die Kraftangriffspunkte auch bei elastischen und leicht plastischen Deformationen praktisch noch am gleichen Ort und kaum von Herstelltoleranzen abhängig. Die Einfederung der durch Schlitze geschwächten Schraubenmutter kann auf diese konstanten Kraftangriffspunkte abgestimmt werden. Zusätzlich zur Reibung zwischen konischen Flächen und Kugelfläche findet auch eine elastische und eine plastische Deformation in Form einer Abplattung statt, die Material im Bereich der Einbuchtungen der Kugelfläche vorstehen lässt und einer Drehung ebenfalls entgegenwirkt. Diese Art der Verbindung gestattet es Knochenschrauben mit und ohne Kragen zu verwenden, Höhenverstellungen am schraubenförmigen Fortsatz der Knochenschraube vorzunehmen und an der eigentlichen Knochenschraube Gewindedurchmesser zu verwenden, die grösser als der Innendurchmesser der Schulter im Aufnahmeteil sind.

Im folgenden wird die Erfindung anhand von Ausführungsbeispielen beschrieben. Es zeigen:
- Fig. 1: Schematisch eine Pedikelschraube mit einem als Schraube ausgebildeten separaten kugelförmigen Kopfteil;
- Fig. 2: schematisch eine Pedikelschraube mit einem als Mutter ausgebildeten separaten kugelförmigen Kopfteil;
- Fig. 3: schematisch eine Standard-Pedikelschraube mit einem als Mutter ausgebildeten separaten kugelförmigen Kopfteil und mit einem verschwenkbaren Aufnahmeteil;
- Fig. 4: schematisch eine Anordnung nach Figur 3 mit fixierter Verbindungsstange;
- Fig. 5: schematisch eine kragenlose Pedikelschraube mit einem in der Höhe verstellbaren kugelförmigen Kopfteil, welches als Mutter ausgebildet ist;
- Fig. 6: schematisch eine Anordnung nach Figur 3, bei der für eine nicht aus der Achse schwenkbare Befestigung Klemmschraube und kugelförmige Mutter durch eine anders dimensionierte Befestigungsmutter ersetzt wurden;
- Fig. 7 bis 14: schematisch jeweils eine Seitenansicht einer mit Schlitz und Eingriffsfläche versehenen kugelförmigen Schraubenmutter und deren Projektionen in Richtung der Schraubenachse;
- Fig. 15: schematisch einen vergrösserten Ausschnitt von Figur 3, aus dem die halben Konuswinkel an Aufnahmeteil und Klemmschraube ersichtlich sind;
- Fig. 16: schematisch den Kopf eines Schraubwerkzeuges, welches für Schraubenmuttern der Figuren 7, 8 und 9 einsetzbar ist; und
- Fig. 17: schematisch in kleinerem Massstab eine Ansicht eines Aufnahmeteils, das mit einer Knochenschraube verbunden ist.

In den Figuren ist ein schwenkbares Befestigungssystem zwischen einer Knochenschraube 1 mit einem kugelförmigen Kopfteil 2 und einem Aufnahmeteil 3 gezeigt. Das Aufnahmeteil besitzt eine Durchgangsbohrung 4 mit einer Schulter am Ende, die das Kopfteil 2 auf seiner Unterseite umfasst und einen Anschlag bildet, wenn das Kopfteil 2 in einer einstellbaren Schwenkstellung 8 durch eine Klemmschraube 7 angepresst wird. Das Kopfteil 2 ist als separates Teil 9 ausgeführt, welches mit der Knochenschraube 1 verschraubbar ist, um das Aufnahmeteil 3 nach dem Implantieren der Knochenschraube 1 aufsetzen und mit dieser verbinden zu können.

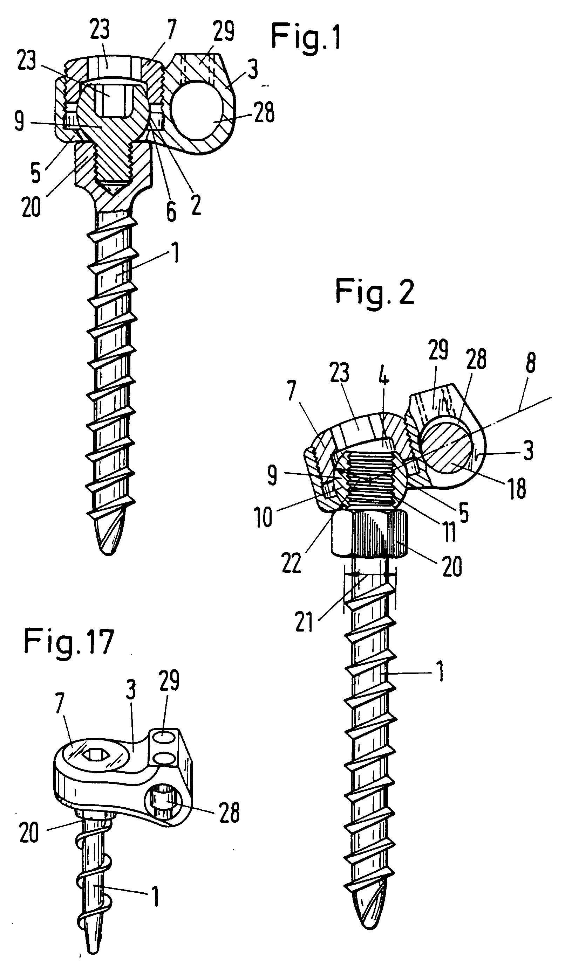

In den Figuren 1, 2, 3, 4, 5, 15 und 17 ist immer wieder ein gleiches Aufnahmeteil 3 gezeigt, das als Befestigungssystem 17 in einer Bohrung 28 eine Verbindungsstange 18 aufnimmt und an dieser mit einer oder zwei Madenschrauben 24 befestigt ist, welche in Gewindebohrungen 29 versenkt sind. Seitlich angeformt ist eine Lasche (Figur 17) mit einer Durchgangsbohrung 4, die quer und versetzt zur Bohrung 28 angeordnet ist und am unteren Ende eine nach innen vorstehende ringförmige Schulter 5 besitzt. Über der Schulter 5 ist die Durchgangsbohrung 4 soweit verbreitert, dass ein kugelförmiger Kopfteil 9, 10 einer Pedikelschraube 1 durch die Schulter 5 gefangen ist und mit einer Klemmschraube 7 an dieser anpressbar ist. Da die Pedikelschrauben 1 mit einem kugelförmigen Kopfteil 2 ausgestattet sind, welches als separates Teil 9 mit der Pedikelschraube 1 verschraubbar ist, bestehen viel mehr Freiheiten in der Formgebung der einzelnen Pedikelschrauben ohne dass auf eine Schwenkbarkeit um einen Kugelkopf verzichtet werden muss.

In Figur 1 ist die Pedikelschraube 1 mit einem Kragen 20 versehen der einen Aussensechskant aufweist, um die Schraube 1 einzudrehen. Der Kragen 20 besitzt eine Bohrung mit Innengewinde, um nach dem Aufsetzen des Aufnahmeteils 3 das separate kugelförmige Kopfteil 9 an einem Innensechskant 23 aufzunehmen und in diese Bohrung einzuschrauben. Das kugelförmige Kopfteil 9 wird gegen einen Anschlag festgezogen der durch ein auslaufendes Gewinde oder die Stirnfläche des Kragens 20 gegeben ist. Wesentlich ist, dass die Schulter 5 mit soviel Spiel durch die Unterseite 6 des Kugelkopfes 2 gefangen ist, dass der vorgesehene Schwenkbereich beim Anliegen der Schulter 5 am Kugelkopf 2 durchfahren werden kann. Die Klemmschraube 7 ist in der Darstellung lose eingeschraubt und sitzt mit einer konischen Anpressfläche auf dem Kugelkopf 2 auf. Die Klemmschraube 7 ist als Deckelschraube mit einem Innensechskant 23 ausgeführt. Statt des Innensechskant 23 könnten auch zwei aus der Achse verschobene Sacklöcher als Angriffsflächen zum Anziehen der Deckelschraube 7 dienen und annähernd geschlossene Räume zwischen Deckelschraube 7 und Schulter 5 schaffen.

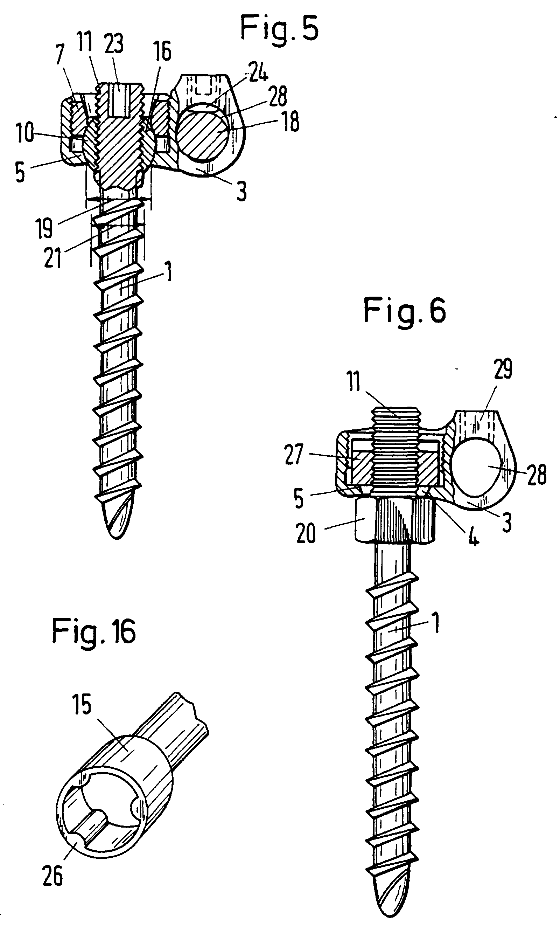

In Figur 2 ist ein gleiches Aufnahmeteil 3 mit einer gleichen Deckelschraube 7 in einer Schwenkstellung 8 auf einem separaten kugelförmigen Kopfteil 9 befestigt, welches als Schraubenmutter 10 ausgeführt ist, die fest mit einem schraubenförmigen Fortsatz 11 der Pedikelschraube 1 verschraubt ist und gegen deren Kragen 20 aufliegt. Das Gewinde der eigentlichen Pedikelschraube 1 hat einen Durchmesser 21, der ohne weiteres grösser als der Innendurchmesser 19 (Figur 5) der Schulter 5 sein darf. Der Kugelradius 22 der Schraubenmutter 10 steht in einer bestimmten Relation zu den Durchmessern der Anpressflächen von Schulter 5 und Klemmschraube 7, die durch die Konuswinkel von mittleren konischen Anpressflächen gegeben ist, wie später gezeigt wird.

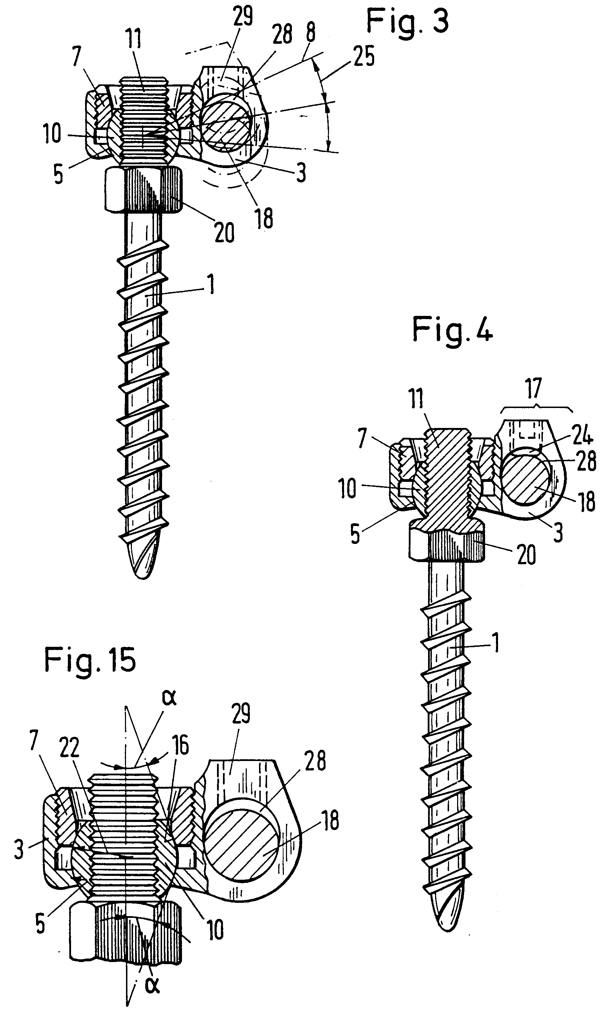

In den Figuren 3, 4 und 15 ist eine StandardPedikelschraube 1 mit einem relativ langen schraubenförmigen Fortsatz 11 gezeigt. In Figur 3 ist die Klemmschraube 7 nur leicht angezogen und erlaubt aus einer mittleren Schwenkstellung Abweichungen 25 der Schwenkstellung 8 für das Aufnahmeteil, die in der Grössenordnung von plus oder minus 15° liegen, wenn, wie in Figur 15 ersichtlich ist, die Anpressfläche der Stützschulter 5 und die Anpressfläche 16 der Klemmschraube 7 konische Flächen mit einem halben Konuswinkel 8° < α < 25° sind. Die konischen Anpressflächen haben den Vorteil, dass die Kraftangriffspunkte am Kugelkopf 10 erhalten bleiben, wenn sich dieser aufgrund einer bewussten Schwächung gegen innen deformiert. Ein halber Konuswinkel α ≈ 20° wird für diesen Fall als vorteilhaft angesehen. Da der schraubenförmige Fortsatz 11 der gezeigten Standard-Pedikelschraube 1 länger als die Höhe vom Kugelkopf 10 ausgeführt ist, wird in der Klemmschraube 11 eine konische Bohrung angebracht, welche die maximal vorgesehene Schwenkung zulässt. Die Klemmschraube 10 ist auf ihrer oberen Seite mit Aussparungen versehen, die das Ansetzen eines Eindrehwerkzeuges erlauben. In Figur 4 ist die Klemmschraube 7 auf der kugelförmigen Schraubenmutter 10 festgesetzt. Solange sich die Schraubenmutter 10 nicht soweit nach innen deformiert, dass das Gewindespiel zwischen Schraubenmutter 10 und dem schraubenförmigen Fortsatz 11 aufgehoben wird, sind einzig die durch das Festziehen der Schraubenmutter 10 auf dem Kragen und Gewinde erzeugten Reibungskräfte dafür verantwortlich, welches Drehmoment bezüglich der Schraubenachse vom Aufnahmeteil 3 auf die Pedikelschraube übertragbar ist.

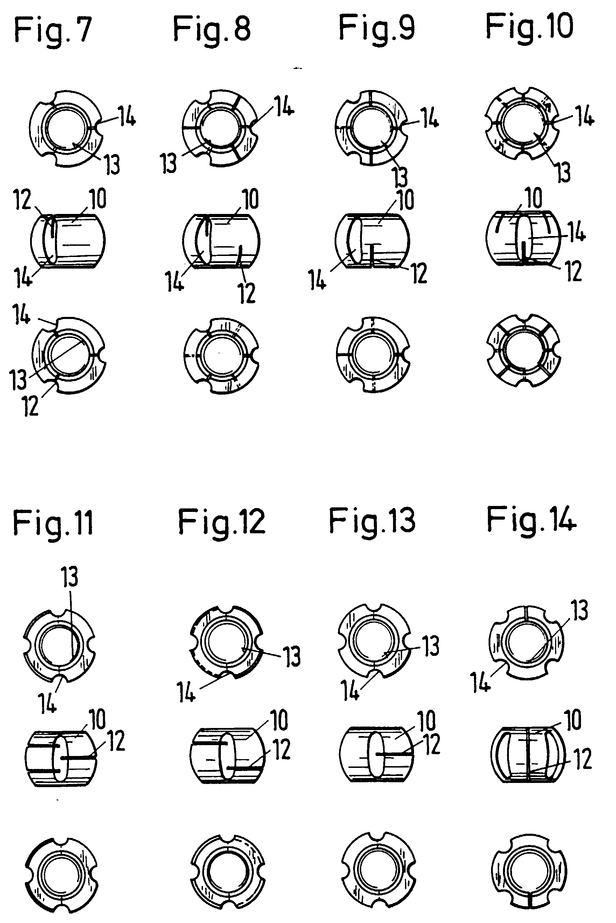

Die Figuren 7 bis 14 zeigen daher eine Vielfalt von künstlich geschwächten kugelförmigen Schraubenmuttern 10, die in diesem Fall durch zusätzliche Schlitze 12 geschwächt sind, welche durch das Gewinde gehen, um beim Zusammenpressen zwischen den konischen Anpressflächen 16, 5 eine Deformation der Gewindegänge nach innen und ein zusätzliches Verklemmen auf dem schraubenförmigen Fortsatz 11 zu erreichen. In den Figuren 7, 8, 9, 10, 14 liegen die Schlitze in Ebenen die durch die Achse der Pedikelschraube gehen. In den Figuren 11, 12, 13 liegen die Schlitze in Ebenen, die senkrecht zur Achse der Pedikelschraube liegen. Andere Schwächungen durch Einstiche und Kerben sind ebenfalls denkbar, wenn sie zu einer Reduktion des Gewindedurchmessers 13 an der kugelförmigen Schraubenmutter 10 führen. An der Aussenseite der Schraubenmutter 10 sind Eingriffsflächen 14 in Form von zylindrischen Ausnehmungen angebracht, die das Eindrehen mit einem Schraubwerkzeug 15 (Figur 16) erlauben, welches passende Vorsprünge 26 aufweist.

In Figur 5 ist eine Verankerung der Schraubenmutter 10 ohne Anschlag gezeigt. Das Eindrehen der Pedikelschraube erfolgt am oberen Ende an einem Innensechskant 23. Nach dem Eindrehen der Pedikelschraube 1, deren Gewindedurchmesser 21 ebenfalls grösser als der Innendurchmesser 19 der Schulter 5 sein darf, kann das Aufnahmeteil 3 aufgesetzt werden. Anschliessend wird die kugelförmige Schraubenmutter 10 auf eine passende Höhe am schraubenförmigen Fortsatz 11 eingeschraubt. Anschliessend wird die Klemmschraube 7 eingeschraubt und das Aufnahmeteil 3 in eine passende Richtung geschwenkt. Mit dem Festziehen der Klemmschraube 7 gegenüber dem Aufnahmeteil 3 setzt sich die deformierbare kugelförmige Mutter 10 auf dem Gewinde fest und wird blockiert. Mit Hilfe der konischen Anpressflächen 16, 5 und der Anzahl und Grösse der Aussparungen 14 können gezielt lokale plastische Deformationen erzeugt werden, die zwischen Schraubenmutter 10 und den Anpressflächen 16, 5 eine starre Verbindung erzeugen.

Um den Baukastencharakter der Erfindung zu unterstreichen ist in Figur 6 gezeigt, wie die Standard-Pedikelschraube 1 und das Aufnahmeteil 3 aus Figur 3 auch direkt mit einer Befestigungsmutter 27 in einer Ebene zueinander bezüglich Drehwinkel einstellbar fixiert werden können.

Die Klemmung der Schulter 5 findet zwischen dem Kragen 20 und der Befestigungsmutter 27 statt.

## Patentansprüche

1. Schwenkbares Befestigungssystem zwischen einer Knochenschraube (1) mit einem kugelförmigen Kopfteil (2) und einem Aufnahmeteil (3), welches am Ende einer Durchgangsbohrung (4) das Kopfteil (2) mit einer Schulter (5) an dessen Unterseite (6) umfasst und welches eine Klemmschraube (7) in der Durchgangsbohrung (4) aufnimmt, mit der das Kopfteil (2) in einer wählbaren Schwenkstellung (8) gegen die Schulter (5) anpressbar ist, dadurch gekennzeichnet, dass das kugelförmige Kopfteil (2) als ein separates Teil (9) ausgeführt ist, welches mit der Knochenschraube verschraubbar ist, um das Aufnahmeteil (3) nach dem Implantieren der Knochenschraube (1) aufsetzen und mit dieser verbinden zu können.

2. Befestigungssystem nach Anspruch 1, dadurch gekennzeichnet, dass das kugelförmige Kopfteil (2) als eine Schraubenmutter (10) ausgeführt ist, die mit einem schraubenförmigen Fortsatz (11) der Knochenschraube (1) verschraubbar ist.

3. Befestigungssystem nach Anspruch 2, dadurch gekennzeichnet, dass die Schraubenmutter (10) durch mindestens einen Schlitz von ihrem Innengewinde (13) gegen aussen geschwächt ist, um das Innengewinde (13) beim Zusammenpressen der kugelförmigen Schraubenmutter (10) durch eine Deformation der Gewindegänge auf dem schraubenförmigen Fortsatz (11) zu fixieren.

4. Befestigungssystem nach Anspruch 2 oder 3, dadurch gekennzeichnet, dass die kugelförmige Schraubenmutter (10) Eingriffsflächen (14) aufweist, die das Ansetzen eines Schraubwerkzeuges (15) ermöglichen.

5. Befestigungssystem nach einem der Ansprüche 1 bis 4, dadurch gekennzeichnet, dass die Schulter (5) und eine Anpressfläche (16) der Klemmschraube (7) als konische Flächen ausgeführt sind.

6. Befestigungssystem nach Anspruch 5, dadurch gekennzeichnet, dass die konischen Flächen einen halben Konuswinkel 8° < α < 25° aufweisen.

7. Befestigungssystem nach Anspruch 6, dadurch gekennzeichnet, dass die konischen Flächen einen halben Konuswinkel α ≈ 20° aufweisen.

8. Befestigungssystem nach einem der Ansprüche 1 bis 7, dadurch gekennzeichnet, dass das Aufnahmeteil (3) eine weitere Befestigung (17) für eine Verbindungsstange (18) aufweist.

9. Befestigungssystem nach einem der Ansprüche 1 bis 9, dadurch gekennzeichnet, dass die Knochenschraube (1) einen über den Innendurchmesser (19) der Schulter (5) nach aussen vorstehenden Kragen (20) aufweist.

10. Befestigungssystem nach einem der Ansprüche 1 bis 10, dadurch gekennzeichnet, dass der Gewindedurchmesser (21) der Knochenschraube (1) grösser ist als der Innendurchmesser (19) der Schulter (5).
